(19)

Europäisches Patentamt

European Patent Office

Office européen des brevets

(11)  **EP 3 407 227 A1**

(12)  **EUROPEAN PATENT APPLICATION**

(43) Date of publication:
**28.11.2018 Bulletin 2018/48**

(51) Int Cl.:
*G06F 19/00* (2018.01)

(21) Application number: **17172689.6**

(22) Date of filing: **24.05.2017**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**MA MD**

(71) Applicant: **Koninklijke Philips N.V.**
**5656 AE Eindhoven (NL)**

(72) Inventors:
• **LANGEREIS, Sander**
**5656 AE Eindhoven (NL)**

• **PLUYTER, Jon Ragnar**
**5656 AE Eindhoven (NL)**
• **KIM, SeYoung**
**5656 AE Eindhoven (NL)**
• **COBBEN, David**
**5656 AE Eindhoven (NL)**

(74) Representative: **de Haan, Poul Erik et al**
**Philips International B.V.**
**Philips Intellectual Property & Standards**
**High Tech Campus 5**
**5656 AE Eindhoven (NL)**

(54)  **MULTI-DISCIPLINARY DECISION SUPPORT**

(57)  The invention relates to clinical decision support tool for supporting a decision based on a plurality of medical findings. The decision to be taken is by a multi-disciplinary team. The medical findings are based on different data sources and integrated to determine a complexity score, based on a consensus and/or conclusiveness of the medical findings. The medical findings and the complexity of the decision are displayed. A circular icon is used to display this information in an intuitive and simple manner. Multiple icons can be combined to arrive at a complexity score of a TNM staging decision.

FIG. 3

EP 3 407 227 A1

**Description**

FIELD OF THE INVENTION

The present invention relates to the field of decision support, and more specifically but not exclusively, to clinical decision support systems (CDSS).

BACKGROUND OF THE INVENTION

Modem healthcare decisions on a patient or subject are often fraught with difficulty and complexity. In the modem era with digital equipment, communications and data, the decision-maker can be swamped with vast amounts of data, which can result in an information overload where the most relevant data might be overlooked or missed.

Furthermore, advances in medical science and knowledge are constantly expanding and are growing rapidly. This scientific explosion has meant that new specialisms have arisen and where medical specialists or experts focus their learning on a deeper understanding of a specific body of medical knowledge.

In some cases, not only have existing medical specialties deepened and narrowed, but new specialist fields are constantly arising. In turn, each of these specialisms is developing and drawing on new diagnostic modalities.

Another issue that often needs to be taken into account is that the health condition of a subject may be multi-faceted and might not be linked to a single measurable or cause. The subject might have several illnesses or afflictions each with its own set of data, diagnoses and treatments - some of which might come into conflict.

There are software tools available to assist medical professions in their decision-making, but these are organized around a specific medical specialty in a "data silo" approach.

There are also user interface systems such as described in US 2006/0242143A1 published on 26 October 2006 that disclose accessing multiple medical images derived from different types of medical imaging systems including at least one repository. A display processor accesses the repository and initiates generation of data representing a composite display image including multiple image windows individually including different medical images derived from corresponding multiple different types of medical imaging systems for a particular anatomic body part of a particular patient.

SUMMARY OF THE INVENTION

According to one aspect of the invention there is provided a device for supporting a decision. The device comprising a receiver for receiving a plurality of findings, a complexity engine for determining a complexity score of the decision based on the findings and a display for displaying the findings and the complexity score. In another embodiment, the findings are medical findings.

According to another embodiment, wherein the plurality of findings are based on corresponding data sources.

According to yet another embodiment, there is a provided a device for supporting a TNM staging decision. The device comprising a receiver for receiving a plurality of medical findings relating to a plurality of lesions, a complexity engine for determining a complexity score of a decision on each lesion based on the medical findings for that lesion, a display for displaying a plurality of icons that indicate whether the decisions for each lesion is conclusive based on the corresponding complexity score, and wherein the complexity engine is further configured to determine an overall complexity score for the TNM staging decision based a number of the icons that are inconclusive.

According to yet another embodiment, the device further comprising a mapping unit for mapping the icons to corresponding anatomical locations of the lesions and wherein the display is configured to display the icons on an anatomical grid.

According to yet another embodiment, there is provided a method for supporting a decision, the method comprising receiving a plurality of findings, determining a complexity score of the decision based on the findings and displaying the findings and the complexity score.

According to yet another embodiment, there is a provided a computer program for enabling a processor to perform the method.

These and other aspects of the invention will be apparent from and elucidated with reference to the embodiments described hereinafter.

BRIEF DESCRIPTION OF THE DRAWINGS

The invention is described by way of example with reference to the following drawings, in which:

Figure 1 shows a device in a first system;
Figure 2 shows a device in a second system;
Figure 3 shows an embodiment of a device for supporting decisions; and
Figure 4a shows an embodiment of a circular icon;
Figure 4b shows a further embodiment of a circular icon with medical findings;
Figure 5 shows a rule engine for determining the complexity of a decision according to one embodiment;
Figure 6 shows an embodiment of mapping of circular icons onto an anatomical grid; and
Figure 7 shows a lung management cancer management system using TNM staging according to an embodiment of the invention.

DETAILED DESCRIPTION OF THE EMBODIMENTS

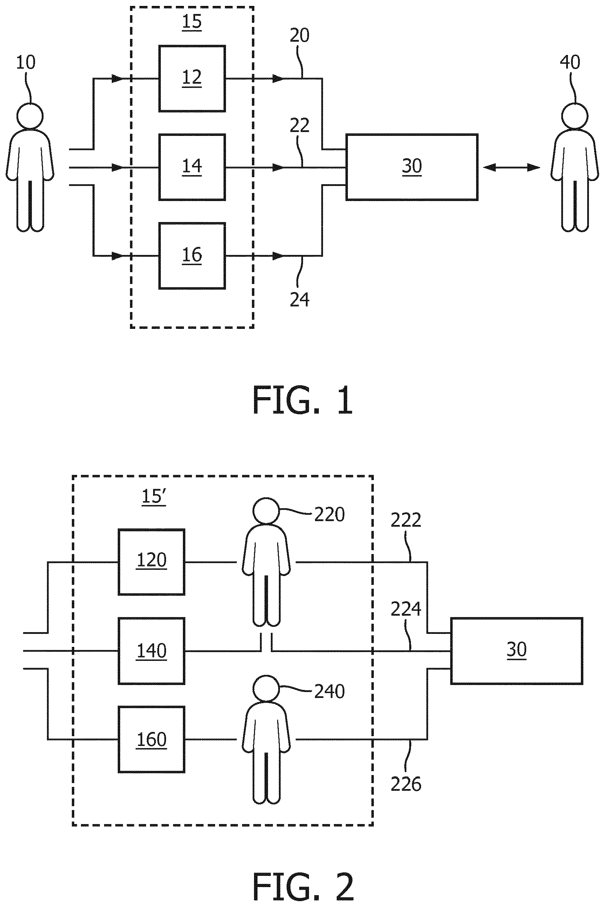

The device 30 and the different systems in which the device may be used, as shown in Figures 1 and 2 respectively, are examples only.

The device is a clinical decision support unit in that it supports the decision of a medical practitioner 40 or a multi-disciplinary team of healthcare professionals. Modern healthcare decisions are often made by a multi-disciplinary team.

The system in Figure 1 is shown to comprise a subject 10 that has a medical condition, which needs to be diagnosed and/or treated by a medical practitioner 40. In the example of Figure 1, the subject 10 has a possible lung tumor and medical staff wish to confirm if it is cancerous.

There are a number of different diagnostic modes (i.e. data sources) that can be used to arrive at a diagnosis. For example, one mode of diagnosis might be histology where a physical biopsy or tissue sample of the lung tumor is taken. The tissue sample is then studied under a microscope or similar equipment for diagnosing the disease in question.

A pathologist is a medical specialist that assesses and interprets the data coming from the histology tests. An endoscopic scan might involve a medical specialist simply giving a lesion a visual scan for irregularities without taking a biopsy. A medical specialist might also take into account the symptoms experienced by the subject, albeit with less of a weighting as compared to the clinical data from the biopsy or samples. The medical specialist might also be for example, an oncologist, pulmonologist, surgeon, etc. depending on tumor type, country, hospital preferences, etc.

Insofar as imaging is concerned, there are also a plurality of imaging diagnostic modes. These might include x-ray, which uses x-rays to get an image of the structure of the internal organs and tissues of the body. A computerized tomography (CT) scan is a series of slices of x-rays analyzed and rendered by a computer to provide a visual display of a lesion from a number of different perspectives. A magnetic resonance imaging (MRI) scan uses magnetic waves rather than x-rays to image a lesion. Ultrasound uses sound or acoustic waves to construct an image of a lesion.

A positron emission tomography (PET) scan fires positively charged particles at a lesion to determine its chemical activity. Unlike MRI, CT and X-ray that are concerned with imaging the structure of a lesion, the PET scan is concerned with imaging the function (chemical and metabolic) of a lesion.

A radiologist is the medical specialist that assesses and interprets the data coming from the various imaging data sources, such as X-ray, PET, CT, ultrasound, etc.

There are also other diagnostic modes where there is an overlap in specialties or where other specialists are needed to interpret the data from other diagnostic mode being used. For example, endoscopic ultrasound (EUS) is a diagnostic mode that combines endoscopy with ultrasound, which allows for visualization of the structure and performing a minimally-invasive biopsy. It requires the skills of a radiologist and a pathologist. Consequently, there is a specialty for assessing and interpreting the data from EUS scans.

Figure 1 shows a number of data sources 12, 14 and 16 from which medical findings 20, 22 and 24 are produced. The data sources 12, 14 and 16 represent the different diagnostic modes or data sources used to diagnose a patient. Figure 2 shows in more detail an example of another system where the data from the data sources 120, 140, 160 is interpreted by a medical specialists 220 and 240. For example, data sources 120 and 140 could represent data from x-rays and PET scans respectively. In the embodiment of Figure 2, the medical specialist 220 is a radiologist. The data source 160 is data from histology or cytology tests, which is interpreted by a different medical specialist 240, who is a pathologist. It will be appreciated that different combinations are possible and that these are examples only. Specialisms can be combined or further differentiated. It will also be appreciated that while Figure 2 is a system where a medical specialist is needed, in yet a further embodiment the interpretative or diagnostic function might be performed by a machine, which is integrated into the data source.

The device 30 receive a plurality of medical findings, shown by lines 20, 22 and 24 in Figure 1 and by lines 222, 224 and 226 in Figure 2. These medical findings might be diagnoses from different specialists based on different data sources. For example, diagnosis 20 resulting from a histology data source 12 is that the lung tumor is malignant (cancerous). However, the diagnosis 22 resulting from an EUS scan data source 14 is that the lung tumor is benign (non-cancerous). Finally, the diagnosis 24 resulting from a PET scan data source 16 is that it is inconclusive from the PET diagnostic mode whether the tumor is malignant or benign. Faced with these different medical findings, the medical practitioner or team 40 needs to try reach consensus on the decision as to the overall diagnosis for the patient.

In one embodiment of the invention, the device 30 is able to receive the medical findings from a plurality of data sources or diagnostic modes. Instead of the device 30 only providing support to single specialty, or based on a single data source, an embodiment of the invention is able to draw on multiple specialties and/or data sources to arrive at a more informed decision as to supporting the diagnosis or treatment of the subject. The advantage of this embodiment is that the medical practitioner 40 is able to draw on a wider range of data to support its decision.

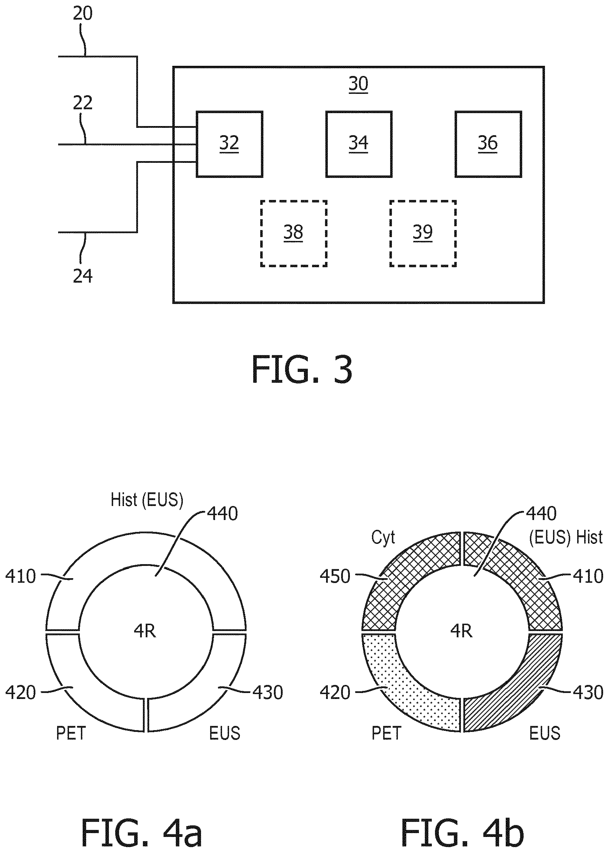

Figure 3 shows an embodiment of the device 30, which comprises a receiving unit 32, a complexity engine 34 and a user interface 36. It also shows that a memory unit 38 and a mapping unit 39 are optionally included. In another embodiment, the memory unit 38 and the mapping unit 39 are not necessary.

The memory unit 38 could be a database for storing medical findings and/or a computer program for implementing the functionality of device 30. More generally, it will also be appreciated by the skilled person that memory, processing and/or any other computing resources, which might be used to implement the various units of the device, could be located remotely from the device 30. For example, cloud-computing could be used to access computing resources remotely over a network. In yet another embodiment, the functions performed by the receiving unit 32, complexity engine 34 and user interface 36 are all combined into one unit.

In one embodiment, the receiving unit 30 is configured to receive the plurality of medical findings 20, 22 and 24 over a wireless link. Alternatively, such medical findings could be communicated over fixed media, such as via cable or fiber optics. In another embodiment the medical findings 20, 22 and 24 are received over the IT network of a hospital, but such medical findings could also be conveyed from more remote locations using the Internet.

In one embodiment, the complexity engine 34 is for determining a complexity score of the decision based on conclusiveness and/or consensus of the medical findings. The user interface 36 has a display for displaying the medical findings and the complexity of the decision. In one embodiment, the displaying of the medical findings and complexity score of the decision is indicated by a circular icon as shown in Figures 4a and 4b.

Specifically, Figures 4a and 4b show a circular icon with medical findings that correspond to three, and four, different data sources respectively. Specifically, Figure 4a shows lymph node 4R of the subject, who has been investigated using histology 410, PET 420 and EUS 430 data sources. Figure 4b shows the same lymph node 4R, but which has additionally been examined using a cytology 450 data source. Figure 4b also shows that the medical findings, which correspond to each of the data sources, are displayed using different fill patterns in corresponding segments of the circular icon. For example, a horizontal fill pattern in segments 410 and 450 indicates that the medical findings based on cytology and histology are malignant. The vertical fill pattern in segment 430 indicates that the medical findings based on EUS are benign. The dotted fill pattern in segment 420 indicates that the medical findings based on PET are inconclusive.

Although Figure 4b shows an embodiment where the segments are differentiated by fill patterns, it will be appreciated this might alternatively be done using gray-scale settings. Such embodiments might be advantageous for black & white or monochrome displays. In yet another embodiment, the segments of the circular icon are color-coded with a red color indicating malignant, a green color indicating benign and a yellow color indicating an inconclusive finding. This embodiment advantageously allows a user to intuitively and simply appreciate what the medical findings are that correspond to each data source, without having to continuously refer to a legend or key to understand their meaning.

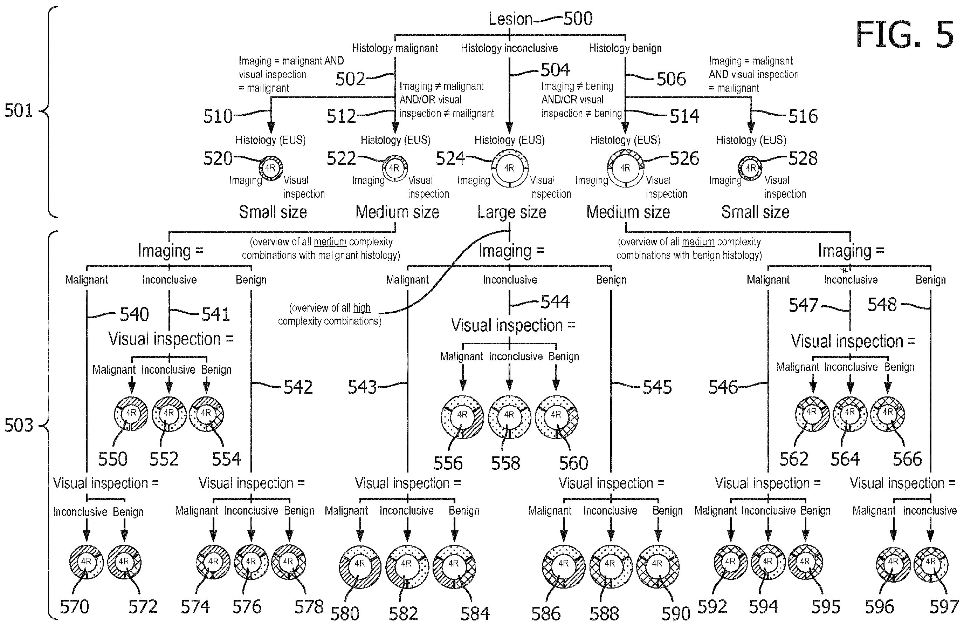

Figure 5 shows a rule engine for determining the size of a circle (or the complexity of a decision) according to one embodiment of the invention. It will be appreciated that other embodiments could implement the functionality of this rule engine using other methods, such as using a look-up table or a weighting function, which are applied to the medical findings.

Figure 5 is an example where a lesion 500 has been investigated using three different diagnostic modes (i.e. three different data sources): histology, imaging and visual inspection. Imaging in this embodiment might mean radiological imaging such as an x-ray, whereas visual inspection might be where a specialist has assessed the lesion with the naked eye for irregularities.

The rule engine of the embodiment in Figure 5 assumes that histology is the most accurate diagnostic mode. In other words, more weight is given to the medical finding relating to histology, as compared to the medical findings based on the other data sources (imaging and visual inspection).

The top-half 501 of Figure 5 shows circle icons 520, 522, 524, 526 and 528 with different sizes. The size of each circle indicates a level of complexity or complexity score of the decision.

For example, a small-sized circle indicates the decision has low complexity, which arises where:

a) the medical findings are conclusive; and
b) there is consensus of the medical findings, which means that the probability of a false positive is low.

Small-sized circles 520 and 528 show the two circumstances when this might arise, which in the case of circle 520 is when all the medical findings indicate that node 4R is malignant, whereas in the case of circle 528 is when all the medical findings indicate that node 4R is benign. A small icon, as displayed on the user interface 36, indicates to a user 40 that this diagnostic decision is reasonably certain. Therefore, not a lot of time needs to be spent by, for example, a multi-disciplinary team analyzing this decision.

A medium-sized circle indicates the decision has medium complexity, which arises where the pathology medical findings are conclusive, but:

a) the medical findings of at least one of the other data sources (imaging or visual inspection) are inconclusive; and/or
b) there is not consensus of the medical findings (i.e. at least one of the medical findings is different). Therefore, the probability of a false positive and a misdiagnosis is medium. For example, if histology is benign (negative) then there is a risk of a false negative, whereas if histology is malignant (positive) then there is a risk of a false positive.

Medium-sized circles 522 and 526 show, with respective lines 540, 541, 542 and lines 546, 547, 548 to the bottom-half 503 of Figure 5, the many different combinations where a medium complexity decision arises. Specifically, circle 522 is where the medical finding based on histology is malignant and the bottom half 503 of Figure 5 shows medium circles 550, 552, 554, 570, 572, 574, 576 and 578 with different combinations of the medical findings based on imaging and visual inspection data. Circle 526 is where the histology based on histology is benign and the bottom half 503 of Figure 5 shows medium circles 562, 564, 566, 592, 594, 595, 596 and 597 with different combinations of the medical findings based on the imaging and visual inspection. These various medium complexity decisions arise when the medical findings based on imaging or visual inspection are inconclusive and/or there is not consensus. A medium icon displayed on the user interface is used to indicate that this decision requires more time for discussion by the multi-disciplinary team, as compared with a small icon, but not as much as compared with a large icon.

A large-sized circle indicates that the decision has high complexity, which arises whenever the medical findings relating to the pathology are inconclusive. This demonstrates the high weighting that is given to pathology as a diagnostic mode for cancer. If the medical finding from pathology is inconclusive, the decision will be high complexity, irrespective of whether or not the medical findings from the other data sources are all conclusive and in consensus. The bottom half of Figure 5 shows various large icons 556, 558, 560, 580, 582, 584, 586, 588 and 590 with different combinations of the medical findings from the imaging and visual inspection data sources. A large icon is used to indicate that these are the most uncertain decisions, which require the most time and discussion from the multi-disciplinary team 40.

It will be appreciated that in a different embodiment, a number can be used to reflect the complexity score of a decision, rather than sizing the circle. The advantage of the embodiment in which the circle is sized, is that it avoids the need for text or numbers or legends, which a user might need to look up. Therefore, the sizing of the icon advantageously contributes to the intuitive and simple manner in which this information is conveyed. In other words, the user is not only being provided with an integrated set of medical findings from a plurality of data sources in a graphical manner, but also the size indicates the complexity score of the decision in a graphical manner that avoids cluttering of the display or overloading the multi-disciplinary team 40 with information.

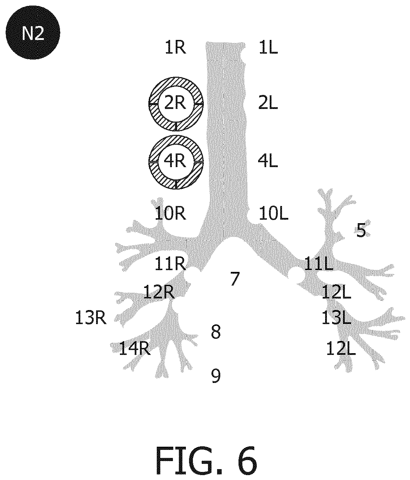

Figure 6 shows the mapping of circular icons onto an anatomical grid. In this embodiment, the mapping unit 39 is needed for locating the circular icons to lesions of the subject's anatomy.

This circular icon embodiment might also be advantageous where it displays a plurality of different sized icons corresponding to different lesions, each loaded with their own dense yet unique information, which is intuitively and efficiently conveyed to a user 40 of the user interface. If the user is multi-disciplinary team, the team can quickly triage which decisions (large circles) they need to spend the most time discussing and why.

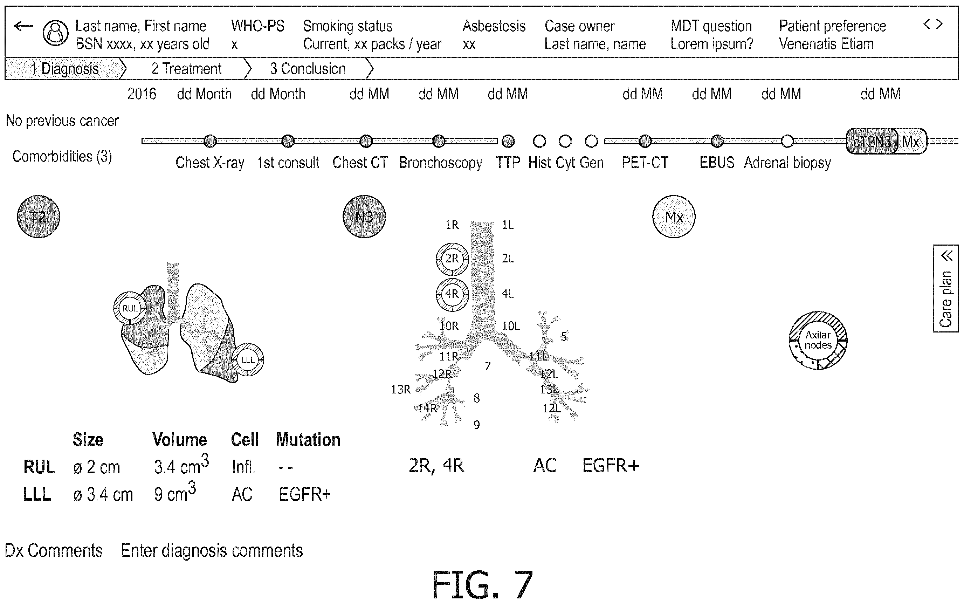

TNM staging describes the extent and level of advancement of a subject's cancer. By identifying the correct TNM staging, the relevant treatment plan can be timed to start as appropriate, such as surgery, chemotherapy and/or radiotherapy. In yet another embodiment, the mapping to the anatomical grid in Figure 6 helps the multi-disciplinary team 40 to derive a TNM stage diagnosis decision based on a set of circular icons representing all data sources relevant to the subject. For example, the involvement of malignant mediastinal lymph nodes (e.g., 2R, 4R) indicates an N2 classification as shown in Figure 6. The anatomical mapping of the mediastinum is used for determining the correct N-stage classification. Figure 7 shows how this is done for T2 (tumor), N3 (node), and Mx (metastases) to help with the TNM staging diagnosis. The current practice is that medical specialists have to make this anatomical mapping in their mind, and hence diagnosing a TNM stage is error prone. The mapping of this embodiment to support the medical specialist in diagnosing the correct TNM staging overcomes these disadvantages.

In another embodiment, the set of circular icons is used to calculate the relative complexity of TNM staging decision. Relative complexity of TNM staging is a function of the number of circular icons and the relative number of inconclusive circular icons. The complexity C of a TNM staging decision according to one embodiment is calculated using the equation:

$$C = x * (1 + ((y + 2z) / x))$$

where:

   $x$ = the number of circular icons that are not conclusively benign or malignant (i.e. total number of icons - low complexity icons)
   $y$ = the number of medium complexity circular icons (i.e indicates a lack of consensus); and
   $z$ = the number of high complexity circular icons (i.e. indicates a lack of consensus with possibly lack of reliable information to make a decision)

Some example scenarios of complexity scores are presented in Table 1 below:

Table 1

| Complexity | x | y | z |
|---|---|---|---|
| 16 | 7 | 5 | 2 |
| 14 | 7 | 7 | 0 |
| 7 | 7 | 0 | 0 |
| 12 | 5 | 3 | 2 |
| 10 | 5 | 5 | 0 |

(continued)

| Complexity | x | y | z |
|---|---|---|---|
| 5 | 5 | 0 | 0 |
| 4 | 1 | 1 | 1 |
| 2 | 1 | 1 | 0 |
| 1 | 1 | 0 | 0 |

An objective measure of relative complexity of TNM staging decision can be utilized a-priory (i.e., before the staging decision is made). This embodiment would therefore advantageously allow the prioritization of cases to be discussed in a tumor board meeting according to the expected complexity of the decision. For example, to start the tumor board meeting with the estimated most complex cases followed by more straightforward cases, or the other way around.

In a different embodiment, parameter x can be utilized during the discussion of the multi-disciplinary team to give a warning when there are inconclusive lesions remaining (i.e., $x > 0$) that need to be addressed before selecting a treatment. For example, in Figure 7 there is still inconclusive information about the adrenal nodes.

While embodiments have been described in an oncology setting, it should be appreciated that this ability to provide a-priory estimates of the complexity of multivariate decisions, applies more broadly to any multi-disciplinary context. Therefore, different embodiments of the invention might deal with other cancer types (e.g., colon, breast, prostate), or other stages in the cancer care path, or other diseases, or even fields other than medicine that require the multi-disciplinary coordination of complex cases, such as military and aviation.

In a further embodiment, there is not only support for diagnostic decisions, but also treatment decisions by integrating other healthcare professions that might be involved in that treatment, for example psychologists, nutrionists, physiotherapists, etc.

The current practice of organizing and assessing data in the "data silo" approach of a single medical specialty, is overcome by an embodiment of the invention that advantageously allows integrating data sources from multiple disciplines for improved decision-making about patient care. This embodiment allows integration of data from different medical domains (e.g. radiology, pathology, oncology, radiotherapy) to guide consensus towards diagnosis and treatment selection in a multi-disciplinary setting.

An embodiment of the invention provides a visually-based user interface for enabling a multi-disciplinary team to make improved decisions by integrating data silos into actionable clinical information, resulting in an increased standard of care.

Another embodiment of the invention integrates data silos and aggregates related and relevant data, according to a logic that is represented in a visual manner, to guide consensus towards TNM stages in a multi-disciplinary setting, which is expected to improve the quality of the diagnosis and treatment selection made by multi-disciplinary teams.

In an embodiment, the functionality of the device 30 is implemented in software, but in other embodiments, the device 30 might be implemented in hardware using, for example, spatially distributed labeled backlit buttons, cards or paper documents.

An embodiment has an icon with a circular shape, but it should be appreciated that other shaped icons (e.g. square, rectangular, triangular, etc.) could also be used. In yet a further embodiment, the upper half of the circular icon represents pathology findings (cytology, histology, etc.), whereas the lower half of the circular icon represents radiological findings (CT, PET, etc.).

According to an embodiment, the device 30 is not for diagnosing or treating the subject, rather to solve a technical problem of how to support medical decisions that need to integrate different medical findings based on different data sources or specialties.

In an embodiment, the plurality of medical findings relating to a decision, based on corresponding data sources selected from a group that includes at least two of following: histology, cytology, visual inspection, x-ray, endoscopic ultrasound, PET, CT, and MRI, ultrasound.

While the invention has been illustrated and described in detail in the drawings and in the foregoing description, such illustration and description are to be considered illustrative and exemplary, not restrictive. The invention is not limited to the disclosed embodiments.

Other variation to the disclosed embodiments can be understood and effected by those skilled in the art in practicing the claimed invention, from a study of the drawings, the disclosure and the appended claims.

In the claims, the word "comprising" does not exclude other elements or steps, and the indefinite article "a" or "an" does not exclude a plurality.

A single unit, processor or other device may fulfill the functions of several items recited in the claims. The mere fact that certain measures are recited in mutually different dependent claims does not indicate that a combination of these features cannot be used to advantage.

Operations like receiving, determining, displaying and/or mapping performed by one or several units can be performed by any other number of units or devices. These operations and/or the control of the decision support system can implemented as program code by means of a computer program and/or as dedicated hardware, firmware or a combination of the foregoing.

A computer program may be stored/distributed on a suitable medium, such as an optical storage medium or a solid-state medium supplied together with or as part of other hardware, but may also be distributed in other forms, such as via the Internet or other wired or wireless telecommunication system. Any reference signs in the claims should not be construed as limiting the scope.

**Claims**

1. A device for supporting a decision, the device comprising:

a receiver for receiving a plurality of findings;
a complexity engine for determining a complexity score of the decision based on the findings; and
a display for displaying the findings and the complexity score.

2. The device of any preceding claim, wherein the plurality of medical findings are based on corresponding data sources.

3. The device of any of claims 1 or 2, wherein the display has an icon for indicating the findings and complexity score.

4. The device of claim 2, wherein the display has a circular icon for indicating:

a type of each data source;
a number of the data sources by splitting the circular icon into a corresponding number of segments;
the findings corresponding to each data source by color-coding the segments; and
the complexity score of the medical score by sizing the circular icon.

5. The device of any preceding claim, wherein the complexity score indicates, to a user of the display, a level of certainty of the decision.

6. The device of any preceding claim, wherein the complexity score is based on conclusiveness and/or consensus of the findings.

7. The device of any preceding claim, wherein the complexity score is based on a weighting applied to the findings on which the decision is based.

8. The device of claim 7, wherein a greater weighting is given to the findings based on pathology as compared to the findings based on other data sources.

9. The device of any preceding claim, wherein each of the findings is a diagnostic decision that a lesion is at least one of malignant, benign or inconclusive.

10. The device of any preceding claim, wherein at least a first of the findings is made by a radiologist and a second of the findings is made by a pathologist.

11. The device of any preceding claim, wherein the decision is a TNM staging decision wherein:

the receiver receives a plurality of medical findings relate to each of a plurality of lesions;
the complexity engine determines a complexity score for a decision on each lesion based on the medical findings relating to that lesion;
the display has a plurality of the icons, each icon indicating whether the decision for the corresponding lesion is conclusive based on the complexity score of the lesion; and
wherein the complexity engine is further configured to determine an overall complexity score of the TNM staging decision based on a number of the icons that are inconclusive.

12. The device of any of claims 3, 4 or 11, further comprising a mapping unit for mapping the icons to corresponding anatomical locations of the lesions and wherein the display is configured to display the icons on an anatomical grid.

13. A method for supporting a decision, the method comprising:

receiving a plurality of findings;
determining a complexity score of the decision based on the findings; and
displaying the findings and the complexity score.

14. The method of claim 13, wherein the plurality of findings are based on corresponding data sources.

15. A computer program for enabling a processor to perform the method of any of claims 13 or 14.

# FIG. 1

# FIG. 2

FIG. 3

FIG. 4a

FIG. 4b

FIG. 5

FIG. 6

| | Last name, First name | WHO-PS | Smoking status | Asbestosis | Case owner | MDT question | Patient preference | |
| ← | BSN xxxx, xx years old | x | Current, xx packs / year | xx | Last name, name | Lorem ipsum? | Venenatis Etiam | < > |

| 1 Diagnosis | 2 Treatment | 3 Conclusion |

2016   dd Month   dd Month   dd MM   dd MM   dd MM   dd MM   dd MM   dd MM   dd MM

No previous cancer

Comorbidities (3)

Chest X-ray   1st consult   Chest CT   Bronchoscopy   TTP   Hist Cyt Gen   PET-CT   EBUS   Adrenal biopsy   cT2N3  Mx

T2     N3     1R   1L     Mx

2R   2L
4R   4L
10R   10L   5
11R   7   11L
12R   12L
13R   8   13L
14R   9   12L

Axilar nodes

| | **Size** | **Volume** | **Cell** | **Mutation** | | | | |
|---|---|---|---|---|---|---|---|---|
| **RUL** | ø 2 cm | 3.4 cm$^3$ | Infl. | - - | | | | |
| **LLL** | ø 3.4 cm | 9 cm$^3$ | AC | EGFR+ | 2R, 4R | AC | EGFR+ | |

Dx Comments   Enter diagnosis comments

Care plan «

# FIG. 7

Europäisches
Patentamt

European
Patent Office

Office européen
des brevets

**EUROPEAN SEARCH REPORT**

Application Number

EP 17 17 2689

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | EP 3 143 534 A1 (KONINKLIJKE PHILIPS NV [NL]) 22 March 2017 (2017-03-22)<br>* page 1, line 1 - page 2, line 29; claim 12; figures 1-5 *<br>* page 4, line 6 - page 5, line 19 *<br>----- | 1-15 | INV.<br>G06F19/00 |
| | | | TECHNICAL FIELDS SEARCHED (IPC)<br><br>G06F |

The present search report has been drawn up for all claims

| Place of search | Date of completion of the search | Examiner |
|---|---|---|
| Munich | 23 November 2017 | Kutzarova, Iskrena |

CATEGORY OF CITED DOCUMENTS

X : particularly relevant if taken alone
Y : particularly relevant if combined with another
   document of the same category
A : technological background
O : non-written disclosure
P : intermediate document

T : theory or principle underlying the invention
E : earlier patent document, but published on, or
   after the filing date
D : document cited in the application
L : document cited for other reasons

& : member of the same patent family, corresponding
   document

EPO FORM 1503 03.82 (P04C01)

**EP 3 407 227 A1**

## ANNEX TO THE EUROPEAN SEARCH REPORT
## ON EUROPEAN PATENT APPLICATION NO.

EP 17 17 2689

This annex lists the patent family members relating to the patent documents cited in the above-mentioned European search report.
The members are as contained in the European Patent Office EDP file on
The European Patent Office is in no way liable for these particulars which are merely given for the purpose of information.

23-11-2017

| Patent document cited in search report | | Publication date | Patent family member(s) | | Publication date |
|---|---|---|---|---|---|
| EP 3143534 | A1 | 22-03-2017 | CN | 106462662 A | 22-02-2017 |
| | | | EP | 3143534 A1 | 22-03-2017 |
| | | | JP | 2017515574 A | 15-06-2017 |
| | | | US | 2017061087 A1 | 02-03-2017 |
| | | | WO | 2015173675 A1 | 19-11-2015 |

EPO FORM P0459

For more details about this annex : see Official Journal of the European Patent Office, No. 12/82

**EP 3 407 227 A1**

**Patent documents cited in the description**

- US 20060242143 A1